# EUROPEAN PATENT SPECIFICATION

(11) **EP 0 935 452 B1**
(45) Date of publication and mention of the grant of the patent: **05.08.2009**
(21) Application number: 97928673.9
(22) Date of filing: 23.05.1997
(51) Int. Cl.: A61L 15/18, A61L 15/46, A61F 13/15

(54) **ABSORBENT ARTICLE HAVING AN ODOUR CONTROL SYSTEM OF ZEOLITE AND SILICA IN CLOSE PHYSICAL PROXIMITY**
ABSORBIERENDE ARTIKEL,DIE EIN GERUCHSKONTROLLSYSTEM VON ZEOLITHEN UND SILIZIUM IN UNMITTELBARER NÄHE ZUEINANDER BESITZEN
ARTICLE ABSORBANT COMPORTANT UN SYSTEME DE LUTTE CONTRE LES ODEURS CONSISTANT EN ZEOLITE ET EN SILICE EN ETROIT VOISINAGE

(30) Priority: 07.06.1996 EP 96109175
(43) Date of publication of application: 18.08.1999
(73) Proprietor: THE PROCTER & GAMBLE COMPANY, Cincinnati, Ohio 45202 (US)
(72) Inventor: GUARRACINO, Mario, I-64028 Silvi Marina (IT); GAGLIARDINI, Alessandro, I-60035 Jesi (IT)
(74) Representative: Briatore, Andrea
(86) International application number: PCT/US1997/008961
(87) International publication number: WO 1997/046188

(56) References cited:
- EP-A- 0 389 015
- EP-A- 0 389 023
- WO-A-81/01643
- US-A- 5 120 693
- US-A- 5 407 442
- US-A- 5 407 442

## Description

### Background of the Invention

The present invention relates to absorbent articles in particular sanitary napkins and panty liners which comprise an odour control system.

### Field of the Invention

Whilst the primary focus of absorbent articles remains the ability of these articles to absorb and retain fluids, another important area of development in this field is the control of odourous compounds contained within the absorbed fluids or their degradation products. There are a wide range of compounds which may be present in an absorbent article during use which result in the formation of malodourous. These compounds include fatty acids, ammonia, amines, sulphur containing compounds and ketones and aldehydes.

The art is replete with descriptions of various odour controlling agents for use in absorbent articles in order to address the problem of malodour formation. These agents can typically be classified according to the type of odour the agent is intended to combat. Odours may be classified as being acidic, basic or neutral. Acidic odour controlling agents have a pH greater than 7 and typically include inorganic carbonates, bicarbonates, phosphates and sulphates. Basic odour controlling agents have a pH of less than 7 and include compounds such as citric acid, boric acid and maleic acid.

The most commonly utilised odour controlling agents are the neutral odour control agents which have a pH of approximately 7. Examples of these known types of compounds include activated carbons, clays, zeolites, silicas, starches and certain combinations thereof. Examples of such agents are described in EPO 348 978 which discloses an absorbent article comprising an odour control system wherein the neutral odour controlling particles are selected from carbon, clays, silicas, zeolites and molecular sieves. Also in EPO 510 619 which relates to an absorbent article comprising odour control complex including a combination of at least 2 agents selected from a group including zeolites and silica gels. Similarly, WO 91/12029. WO 91/11977 and WO 91/12030 disclose certain combinations of zeolites and absorbent gelling materials.

WO 81/01643 relates to the removal of nitrogenous irritants present in waste matter in diapers by the use of an inorganic aluminosilicate zeolite ammonium ion exchange material. In addition to the optional presence of silica gel to absorb water, activated carbon is a preferred component of the odour control system.

US 5407442 describes coated carbon particles combined with white odor controlling agents.

Many of these neutral odour control agents however have certain associated disadvantages. Silica and silica molecular sieves are considered expensive odour control agent components. Also activated carbon which has been noted in the art as providing particularly effective odour control it is not favoured due to its black appearance, which is considered unacceptable by consumers. Zeolites which unlike carbon do not have a negative aesthetic profile are not considered to deliver effective odour control over a broad range of odour types. Moreover, another disadvantage is that the more effective types of zeolites, the so-called intermediate and high ratio SiO₂/AlO₂ zeolites are particularly expensive. Furthermore, zeolites often require the use of specific manufacturing or processing techniques to ensure the required particle size so as to minimize dust formation and to ensure a homogeneous distribution of the zeolite within the absorbent article.

A further problem also exits when utilising multi-component odour control systems, particularly those comprising zeolite. In such systems, it is essential that the components are blended prior to their incorporation into the absorbent article. This is in order that the odour control system can be satisfactorily homogeneously incorporated within the absorbent article at the desired location.

Hence, there still exists a need to provide an odour controlling agent or system which has an acceptable aesthetic profile, such that it is light coloured and which provides effective odour control over a wide range of malodourous compounds. In particular, there is a need to provide an odour control system which comprises readily available, inexpensive materials which may be homogeneously incorporated into absorbent articles without the necessity of pre-blending or further processing.

It has now been observed that these needs may be addressed by the use of zeolites, particularly the intermediate and lower zeolites, which are in close physical proximity with silica, in the form of an agglomerate or particulate within the absorbent article.

### Summary of the Invention

The present invention relates to an absorbent articles comprising a liquid pervious topsheet, a backsheet and an absorbent core intermediate said topsheet and said backsheet and an odour control system, characterised in that said odour control system comprises a zeolites and silica wherein silica is in a highly purified form containing at least 90% silicon dioxide and, wherein said zeolite and silica are in close physical proximity within a particulate, granulate, flake, noodle or extrudate.

### Detailed Description of the Invention

### Odour control system

According to the present invention the absorbent article comprises as an essential feature an odour control system comprising zeolite and silica, which is effective over a wide range of malodours wherein the zeolite and silica are in close physical proximity.

### Close physical proximity

According to the present invention the odour control system comprises zeolite and silica which are in close physical proximity within said absorbent articles. Close physical proximity as used herein encompasses particulates, granulates, flakes, noodles and extrudates containing said zeolite and said silica. In a preferred embodiment of the present invention said zeolite and said silica are in intimate admixture within said composition such that they are adjacent within said particulate, granulate, flake, noodle and extrudate. In another embodiment of the present invention the zeolite and said silica are present in the same particulate, granulate, flake, noodle or extrudate, but are not adjacent and are separated by one or more of the optional additional components of the particulate, flake, granulate, noodle or extrudate, for example by means of at least one layer.

The odour control system is manufactured in a conventional manner, utilising spray drying, spray mixing or agglomeration processes.

The mean particle size of the particles of the odour control system in accordance with the invention should preferably be such that no more than 5% of the particles are greater than 1.7mm in diameter and not more than 5% are less than 0.15mm in diameter. Preferably the mean particle size is from 0.1 mm to 2mm, preferably from 0.2mm to 0.7mm, most preferably from 0.3mm to 0.5mm.

### Zeolite odour control agent

The use and manufacture of zeolite material is well know in the literature and is described in the following reference texts: ZEOLITE SYNTHESIS, ACS Symposium Series 398, Eds. M. L. Occelli and H. E Robson (1989) pages 2-7; ZEOLITE MOLECULAR SIEVES, Structure, Chemistry and Use, by D. W. Breck, John Wiley and Sons (1974) pages 245-250, 313-314 and 348-352; MODERN APPLICATIONS OF MOLECULAR SIEVE ZEOLITES, Ph.D. Dissertation of S. M. Kuznicki, U. of Utah (1980), available from University of Microfilms International, Ann Arbor, Michigan, pages 2-8.

Zeolites are crystalline aluminosilicates of group IA and group IIA elements such as Na, K, Mn, Ca and are chemically represented by the empirical formula

M_{2/n}O. Al₂O₃. ySiO₂ . wH₂O

where y is 2 or greater, n is the cation valence, and w is the water content in the voids of the zeolite.

Structurally, zeolites are complex, crystalline inorganic polymers based on an infinitely extending framework of AlO₄ and SiO₄ tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

M_{x/n} [(AlO₂)x (SiO₂)_{y}]. wH₂O

where n is the valence of cation M, w is the number of water molecules per unit cell, x and y are the total number of tedrahedra per unit cell, y/x usually having values of 1-5.

Zeolites may be naturally derived or synthetically manufactured. The synthetic zeolites being preferred for use herein. Suitable zeolites for use herein include zeolite A, zeolite P, zeolite Y, zeolite X, zeolite DAY, zeolite ZSM-5, or mixtures thereof. Most preferred are zeolite A, zeolite Y or mixtures thereof.

According to the present invention the zeolite is preferably hydrophobic. This is typically achieved by increasing the molar ratio of the SiO₂ to AlO₂ content such that the ratio of x to y is at least 1, preferably from 1 to 500. most preferably from 1 to 6.

The absorbent article preferably comprises from 40gm² to 90gm², more preferably from 55gm² to 85gm², most preferably from 60gm² to 65gm² of said zeolite.

### Silica odour control agent

According to the present invention the odour control system comprises as an essential component silica in combination with the zeolite. Silica i.e. silicon dioxide SiO₂ exists in a variety of crystalline forms and amorphous modifications and can be derived from both natural sources such as diatomaceous earth and synthetic sources, any of which are suitable for use herein. In particular, silicas having a high surface area or in agglomerated form are preferred. Silica molecular sieves are not considered to be within the definition of silica as used herein. The silica is in a highly purified form such that is contains at least 90%, preferably 95%, more preferably 99% silicon dioxide. Most preferably the silica is silica gel having a 100% silica content. Alternatively, the silica may be provided from other sources such as metal silicates including sodium silicate.

The absorbent article preferably comprises from 40gm⁻² to 100gm⁻², more preferably from 60gm⁻² to 90gm⁻², most preferably from 60gm⁻² to 65gm⁻² of silica based on 100% purity.

The silica (100%) and zeolite are preferably present in the odour control system at a ratio by weight of from 1:5 to 5:1, more preferably from 3:1 to 1:3, most preferably from 1:1. Typically, the odour control system comprises 90% to 5% by weight zeolite and from 5% to 90% by weight silica.

According to the present invention the weight of the odour control system which may be used in the absorbent article can be readily determined by the skilled person bearing in mind the absorbent article dimensions. For example the absorbent article may comprise from 0.5g to 5g, preferably from 1g to 3g, most preferably from 1.5g to 2.5g of said odour control system.

According to the present invention the odour control system may comprise additional optional components such as absorbent gelling materials, antimicrobial agents, perfuming ingredients, masking agents, activated carbon and chelants, such as for example ethylene diaminetetraacetic acid (EDTA) all of which are known to the those skilled in the art. Particularly preferred are absorbent gelling materials and EDTA.

In addition to the silica which in itself acts as a binder at least when present as a silicate, the odour control system may comprise as optional components additional binder materials. Any binder materials used in agglomeration or spray drying techniques may be used herein for example starches, cellulose, gums, anionic and nonionic surfactants such as PEG, fatty acids, fatty alcohols, ethoxylates, EDTA or any mixtures thereof.

Preferred binder materials are well known in commerce under various trade names such as GELFORM, PURAGEL, LAVERAL, MALTRIN and METHOCEL. In general these binders are soluble or dispersible in water or body fluids such as blood and urine. Chemically such preferred binders comprise various starch, cellulose, modified starch, modified cellulose, gum acacia, gum arabic, soluble gelatine materials or mixtures thereof. Carboxymethylcellulose and hydroxypropylcellulose are more preferred binders for use herein. Typically, the above described binder will comprise at least 1% to 50%, preferably from 3% to 15%, most preferably from 4% to 10% by weight of the final particles, agglomerates or aggregates of the odour control system produced herein. The amount of binder used can be readily determined by the skilled man and will depend on the binder material used and the desired particle size of the odour control system.

The odour control system may be incorporated into the absorbent article by any of the methods disclosed in the art, for example layered on the core of the absorbent article or mixed within the fibres of the absorbent core. The odour control system is preferably incorporated between two layers of cellulose tissue. Optionally the system may be bonded between two cellulose tissue layers with, for example, a hot melt adhesive or any suitable bonding system.

More preferably the odour control system is incorporated in a layered structure in accordance with the disclosure of WO 94/01069 or Italian patent application number TO 93A 001028 (WO 95/17868). TO 93A 001028 (WO 95/17868) describes a layered structure substantially as described in WO 94/01069 with the exception that TO 93A 001028 (WO 95/17868) comprises a much higher quantity of absorbent gelling material (AGM) in the intermediate layer which is between the fibrous layers (120gm⁻²) that would be incorporated as an optional component in the present invention. The intermediate layer comprises in particular a polyethylene powder as thermoplastic material which is mixed with the odour control system of the present invention. The mixture is then heated such that the polyethylene melts and glues the laminate layers and components together. The bridges which form the bond points between the fibrous layers involve particles of AGM as well as particles of thermoplastic material. (The absorbent capacity of the AGM is unaffected by bonding.) The adhesive lines are preferably also placed on the edges of the laminate to ensure that the edges of the laminate stick and any loose odour control material does not fall out of the laminate.

### Absorbent article

According to the present invention the absorbent article comprises a topsheet, backsheet and absorbent core and may in addition comprise additional features such as wings or fastenings depending on the end use of the product.

### Absorbent core

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent core may have any thickness depending on the end use envisioned.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orion), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### The topsheet

According to the present invention the absorbent article comprises as an essential component a topsheet The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as woven and non woven fabrics and films. In a preferred embodiment of the present invention at least one of the layers, preferably the upper layer, of the topsheet comprises a hydrophobic, liquid permeable apertured polymeric film. Preferably, the upper layer is provided by a film material having apertures which are provided to facilitate liquid transport from the wearer facing surface towards the absorbent structure. If present the lower layer preferably comprises a non woven layer, an apertured formed film or an airlaid tissue.

### Backsheet

The backsheet primarily prevents the extrudes absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film.

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matt finished to provide a more clothlike appearance. Further, the backsheet can permit vapours to escape from the absorbent structure, i.e. be breathable, while still preventing extrudates from passing through the backsheet. Also breathable backsheets comprising several layers, e.g. film plus non-woven structures, can be used.

According to the present invention the absorbent article may find utility in sanitary napkins, panty liners, adult incontinence products and baby diapers. In particular the present invention finds application in sanitary napkins and panty liners.

### Processing

According to the present invention the intimate admix of zeolite and silica may be prepared by any method known in the art such as by agglomeration, spray drying techniques or fluid bed coating. Particularly preferred are agglomeration methods.

### Agglomeration

Accordingly, the agglomeration process steps of the present invention comprise:
1. Mixing
2. Transferring to a high speed mixer.
3. Agglomerating.
4. Drying

It will be understood that any convenient order of the process steps listed above can be contemplated. Also it may be possible and even advantageous to carry out two or more of the above operations in a single piece of process equipment. Each of these operations will now be described in more detail.
1. Making a Paste Premix: The paste premix may be prepared by any method which is known to the man skilled in the art. In the present invention the binder is mixed with water and the optional ingredients. This may be achieved in any convenient piece of mixing equipment, and may be carried out using any order of addition of the separate or pre-mixed components.
2. & 3. Fine Dispersion Mixing and Granulation: Any apparatus, plants or units suitable for the processing of surfactants can be used for carrying out the process according to the invention. For mixing/ agglomeration of the silica and zeolite with the paste any of a number of mixers/agglomerators can be used, particularly high and middle shear mixers. In one preferred embodiment, the process of the invention is continuously carried out. Especially preferred are mixers of the Fukae^{R} FS-G series manufactured by Fukae Powtech Kogyo Co., Japan; this apparatus is essentially in the form of a .bowl-shaped vessel accessible via a top port, provided near its base with a stirrer having a substantially vertical axis, and a cutter positioned on a side wall. The stirrer and cutter may be operated independently of one another and at separately variable speeds. The vessel can be fitted with a cooling jacket or, if necessary, a cryogenic unit.

Other similar mixers found to be suitable for use in the process of the invention include Diosna^{R} V series ex Dierks & Söhne, Germany; and the Pharma Matrix^{R} ex T K Fielder Ltd., England. Other mixers believed to be suitable for use in the process of the invention are the Fuji^{R} VG-C series ex Fuji Sangyo Co., Japan; and the Roto^{R} ex Zanchetta & Co srl, Italy.

Other preferred suitable equipment can include Eirich^{R}, series RV, manufactured by Gustau Eirich Hardheim, Germany; Lödige^{R}, series FM for batch mixing, series Baud CB/KM for continuous mixing/agglomeration, manufactured by Lödige Machinenbau GmbH, Paderborn Germany; Drais^{R} T160 series, manufactured by Drais Werke GmbH, Mannheim Germany; and Winkworth^{R} RT 25 series, manufactured by Winkworth Machinery Ltd., Berkshire, England.

The paste can be introduced into the mixer at an initial temperature between its softening point (generally in the range of 40-60°C) and its degradation point (depending on the chemical nature of the paste. High temperatures reduce viscosity simplifying the pumping of the paste but result in lower active agglomerates.

The introduction of the paste into the mixer can be done in many ways, from simply pouring to high pressure pumping through small holes at the end of the pipe, before the entrance to the mixer. The method utilised depending principally on the binder material used. If necessary, extrusion of the paste and/or pumping pressures prior to the entrance in the mixer may be utilised.
4. Drying: It is also within the scope of the present invention that the resulting granules may be dried, for example, using a fluidized bed or cooled and/or dusted with a suitable surface coating agent.

### Spray drying process

According to the present invention the intimate admixture of zeolite and silica may also be prepared using spray drying methods known in the art. Using such a process a mixture of silica, zeolite and binder is mixed in a vessel with stirring. Water is added along with optional viscosity modifying agents. The pH is adjusted to be greater than 10 and additional water is added in order to provide the mixture with the desired flow characteristics. The mixture is stirred for up to 1 hour. The mixture is then transferred to the top of a spray drying tower unit. The inlet temperature and the resonance time is varied to produce the desired particle size and moisture content. The resulting spray dried powder is collected at the base of the tower.

### Examples:

The sanitary napkins used in the following examples were Always (Always is a registered Trade Mark) as sold by the Procter & Gamble Company. Each napkin was opened by cutting the wrap around the perforated coverstock at its bottom face approximately along a longitudinal edge of the release paper which covers the external adhesive layer. The side of the absorbent fibrous core is then exposed by slightly shifting the water impermeable plastic bottom layer and subsequently, the fibrous core is split into two halves, each having approximately the same thickness, along a plane which is parallel to the plane of the napkin itself. The particulate/agglomerated odour control system is homogeneously distributed between these two fibrous layers which are then joined together to reconstitute the absorbent core.

The water impermeable inner backsheet is then put back into its original position and the wrap around perforated coverstock is sealed along the cut by means of a e.g. a double sided adhesive tape.

Samples were produced using the method above, containing the odour control systems as described hereinbelow. A commercially available Always sanitary napkin without modification was used as a reference.

The zeolite used is zeolite A, Wessalith CS, available from Degussa AG. The silica used is Syloblanc 82 available from Grace GmbH or FK700 from Degussa. The binder used is carboxymethylcellulose.

| Odour control system | Zeolite (g/napkin) | Silica (g/napkin) | Binder | Close physical proximity |
|---|---|---|---|---|
| Reference 0 | 0 | 0 | 0 | N/A |
| Reference 1 | 0 | 0.96 | 0.4 | Yes (Agglomerate) |
| Reference 2 | 0.5 | 0.5 | 0 | No -(blended) |
| Product 1 | 0.48 | 0.48 | 0.4 | Yes (Agglomerate) |

The above odour control systems were tested using the odour control test outlined below.

### Odour control test

### Obiective of the test

In-vitro sniff test is an analytical method for evaluating the malodor adsorbent power of test compounds when in contact with a source of malodors.

### Principle of the test

The in-vitro sniff test consists of putting in contact the samples with malodorant fluids and sniffing and grading the unpleasantness of the malodor.

### Execution of the test

1,0 g of test compound is placed into a glass evaporating dish (capacity 20 ml). 0.5 ml of malodorant fluid are added into the dish by using a graduate pipette. The total content of the evaporating dish is homogeneously mixed by using a glass rod. The evaporating dish is covered with an aluminum sheet (with 20 holes of about 1 mm diameter) and after 20 minutes odor is evaluated by three expert sniffers.

### Malodorant fluids

Any fluid commonly utilized for malodour testing in sanitary articles such as menstrual fluid, artificial menstrual fluid or malodor treated paper industry fluid (PIF) may be used for the test herein described.

PIF comprises (NaCl (1.0g.), Carboxymethylcellulose(1.5g.), 8g Glycerol (8g), NaHCO₃ (0.4g) in 100 ml of H₂O) and a specific malodourous chemicals e.g. 0.6 g butyric acid or 0.5 ml trimethylamine.

### Sniff test

Sniff test session takes place in a large air-conditioned room with relatively rapid air turnover and is performed by at least six graders who have to sniff all the products of the same woman in each sniff test session. The grader may use any convenient sniffing strategy during this time, but is asked to be consistent throughout the test- During a test, graders sniff on the perforated aluminium sheet for approximately 5 seconds; the graders sniffs products at several seconds intervals from each them. In these conditions every sniffer evaluates the odour of each series of products using a (Un)pleasantness scale which ranges from -10 (highest level of unpleasantness) to 5 (most pleasant). With this procedure, each grader compares MU (Unpleasantness) in the test session. The relative MU odour values from different products are assigned numbers. For example, in a test session, a sample that is perceived to be twice as strong as another is assigned twice as large a number. One that perceived to be one-tenth as strong as another is assigned a number one-tenth as large, etc. In each test session, zero is used to designate neutral hedonicity, and + and - numbers are assigned in ratio proportion to the relative pleasantness and unpleasantness of the odour.

The Unpleasantness values, for each sample, is obtained as a mean of at least 72 observations (two products each, 3 graders).

### Statistical analysis of the data

The results collected from the test is then analysed by statistical analysis software (SAS). The data is processed to show statistically significant differences among untreated and treated products. The difference is shown in the tables by means of a letter near every mean value. Results with the same letter are not statistical significantly different. Duncan's multiple range test is used to form multiple comparisons.

### Results

Using the above method values of the (Un)pleasantness of the odour (MU) are obtained. Generally MU values are negative i.e. the higher are the negativity the stronger the unpleasantness of the odour. The MU value gives an indication of the effectiveness of an odour control system.

From the test resulted that the product 1 which comprises 4% less active component than reference 2, not only provides the same odour control performance as reference 2, but surprisingly further improves the performance by 12% thereover.

## Claims

1. An absorbent article comprising a liquid pervious topsheet, a backsheet and an absorbent core intermediate said topsheet and said backsheet and an odour control system, **characterised in that** said odour control system comprises a zeolite and silica, wherein silica is in a highly purified form containing at least 90% silicon dioxide, and wherein said zeolite and silica are in close physical proximity within a particulate, granulate, flake, noodle or extrudate.

2. An absorbent article according to claim 1, wherein the ratio of said zeolite to said silica is from 1:5 to 5:1.

3. An absorbent article according to any one of the preceding claims, wherein said zeolite has a ratio of SiO₂ to AlO₂ of at least 1.

4. An absorbent article according to any one of the preceding claims, wherein said zeolite has a ratio of SiO₂ to AlO₂ of from 1 to 500.

5. An absorbent article according to any one of the preceding claims, wherein said zeolite is zeolite A.

6. An absorbent article according to any one of the preceding claims, wherein said article comprises from 0.5g to 5g of said odour control system.

7. An absorbent article according to any one of the preceding claims, wherein said odour control system comprises an agglomerate comprising said zeolite and said silica.

8. An absorbent article according to claim 1, wherein said particulate further comprises adjuncts selected from additional odour control agents, binder materials or mixtures thereof.

9. An absorbent article according to claim 8, wherein said odour control system comprises at least 1% of said binder material.

10. An absorbent article according to claim 9, wherein said odour control system comprises from 3% to 15% of said binder material.

11. An absorbent article according to claim 8, wherein said binders are selected from starch, cellulose, modified starch, modified cellulose, gum acacia, gum arabic, soluble gelatine materials, ethylene diaminetetraacetic acid or mixtures thereof.

12. An absorbent article according to claim 1, wherein said particulates, granulates, flakes, noodles or extrudate has an average diameter of from 0.1 mm to 2 mm.

13. An absorbent article according to any one of the preceding claims, wherein said article is a sanitary napkin or a panty liner.

## Patentansprüche

1. Absorptionsartikel, umfassend eine flüssigkeitsdurchlässige Oberschicht, eine Unterschicht und einen Absorptionskern zwischen der Oberschicht und der Unterschicht und ein Geruchsbekämpfungssystem, **dadurch gekennzeichnet, dass** das Geruchsbekämpfungssystem einen Zeolith und Silica umfasst, wobei Silica in einer hochreinen Form vorliegt, die zu mindestens 90 % Siliciumdioxid enthält, wobei der Zeolith und Silica in enger physischer Nähe innerhalb eines Teilchens, eines Granulats, einer Flocke, einer Nudel oder eines Extrudats vorliegen.

2. Absorptionsartikel nach Anspruch 1, wobei das Verhältnis von dem Zeolith zu dem Silica von 1:5 bis 5:1 beträgt.

3. Absorptionsartikel nach einem der vorstehenden Ansprüche, wobei der Zeolith ein Verhältnis von SiO₂ zu AlO₂ von mindestens 1 aufweist.

4. Absorptionsartikel nach einem der vorstehenden Ansprüche, wobei der Zeolith ein Verhältnis von SiO₂ zu AlO₂ von 1 bis 500 aufweist.

5. Absorptionsartikel nach einem der vorstehenden Ansprüche, wobei der Zeolith Zeolith A ist.

6. Absorptionsartikel nach einem der vorstehenden Ansprüche, wobei der Artikel 0,5 g bis 5 g des Geruchsbekämpfungssystems umfasst.

7. Absorptionsartikel nach einem der vorstehenden Ansprüche, wobei das Geruchsbekämpfungssystem ein Agglomerat umfasst, das den Zeolith und das Silica umfasst.

8. Absorptionsartikel nach Anspruch 1, wobei das Teilchen ferner Zusatzstoffe umfasst, die aus zusätzlichen Geruchsbekämpfungsmitteln, Bindemitteln oder Mischungen davon ausgewählt sind.

9. Absorptionsartikel nach Anspruch 8, wobei das Geruchsbekämpfungssystem zu mindestens 1 % das Bindemittel umfasst.

10. Absorptionsartikel nach Anspruch 9, wobei das Geruchsbekämpfungssystem zu 3 % bis 15 % das Bindemittel umfasst.

11. Absorptionsartikel nach Anspruch 8, wobei die Bindemittel aus Stärke, Cellulose, modifizierter Stärke, modifizierter Cellulose, Akaziengummi, Gummiarabikum, löslichen Gelatinematerialien, Ethylendiamintetraessigsäure oder Mischungen davon ausgewählt sind.

12. Absorptionsartikel nach Anspruch 1, wobei die Teilchen, Granulate, Flocken, Nudeln oder das Extrudat einen durchschnittlichen Durchmesser von 0,1 mm bis 2 mm aufweisen.

13. Absorptionsmittel nach einem der vorstehenden Ansprüche, wobei der Artikel eine Damenbinde oder eine Slipeinlage ist.

## Revendications

1. Article absorbant comprenant une feuille de dessus perméable aux liquides, une feuille de fond et une âme absorbante entre ladite feuille de dessus et ladite feuille de fond et un système de régulation des odeurs, **caractérisé en ce que** ledit système de régulation des odeurs comprend une zéolite et de la silice, dans lequel la silice est sous une forme hautement purifiée contenant au moins 90 % de dioxyde de silicium, dans lequel la zéolite et la silice sont en proximité physique étroite au sein d'une matière particulaire, une matière granulaire, une paillette, une nouille ou un extrudat.

2. Article absorbant selon la revendication 1, dans lequel le rapport de ladite zéolite sur ladite silice va de 1:5 à 5:-1.

3. Article absorbant selon l'une quelconque des revendications précédentes, dans lequel ladite zéolite a un rapport de SiO₂ sur AlO₂ d'au moins 1.

4. Article absorbant selon l'une quelconque des revendications précédentes, dans lequel ladite zéolite a un rapport de SiO₂ sur AlO₂ allant de 1 à 500.

5. Article absorbant selon l'une quelconque des revendications précédentes, dans lequel ladite zéolite est la zéolite A.

6. Article absorbant selon l'une quelconque des revendications précédentes, où ledit article constitue de 0,5 g à 5 g dudit système de régulation des odeurs.

7. Article absorbant selon l'une quelconque des revendications précédentes, dans lequel ledit système de régulation des odeurs comprend un agglomérat comprenant ladite zéolite et ladite silice.

8. Article absorbant selon la revendication 1, dans lequel ladite matière particulaire comprend, en outre, des additifs choisis parmi des agents de régulation des odeurs supplémentaires, des matériaux de type liant ou leurs mélanges.

9. Article absorbant selon la revendication 8, dans lequel ledit système de régulation des odeurs comprend au moins 1 % dudit matériau de type liant.

10. Article absorbant selon la revendication 9, dans lequel ledit système de régulation des odeurs comprend de 3 % à 15 % dudit matériau de type liant.

11. Article absorbant selon la revendication 8, dans lequel lesdits liants sont choisis parmi l'amidon, la cellulose, l'amidon modifié, la cellulose modifiée, la gomme d'acacia, la gomme arabique, des matériaux à base de gélatine soluble, de l'acide éthylène diamine tétra-acétique ou leurs mélanges.

12. Article absorbant selon la revendication 1, dans lequel lesdites matières particulaires, granulés, paillettes, nouilles ou extrudats ont un diamètre moyen allant de 0,1 mm à 2 mm.

13. Article absorbant selon l'une quelconque des revendications précédentes, où ledit article est une serviette hygiénique ou un protège-slip.
